# EUROPEAN PATENT SPECIFICATION

(11) **EP 3 611 135 B1**
(45) Date of publication and mention of the grant of the patent: **16.06.2021**
(21) Application number: 18197043.5
(22) Date of filing: 27.09.2018
(51) Int. Cl.: C02F 1/24, C02F 1/52, C02F 1/66, C02F 1/68, C02F 3/28, C02F 3/30, C02F 9/14, C02F 9/02, C02F 9/04

(54) **DEVICE AND METHOD FOR PHARMACEUTICAL WASTEWATER TREATMENT WITH HIGH EFFICIENCY RESOURCE RECOVERY AND LOW ENERGY CONSUMPTION**
VORRICHTUNG UND VERFAHREN ZUR PHARMAZEUTISCHEN ABWASSERBEHANDLUNG MIT HOHER EFFIZIENZ DER RESSOURCENRÜCKGEWINNUNG UND NIEDRIGEM ENERGIEVERBRAUCH
DISPOSITIF ET PROCÉDÉ DE TRAITEMENT DES EAUX PHARMACEUTIQUES USÉES À RÉCUPÉRATION DE RESSOURCES À HAUT RENDEMENT ET À FAIBLE CONSOMMATION D'ÉNERGIE

(30) Priority: 15.08.2018 CN 201810930132
(43) Date of publication of application: 19.02.2020
(73) Proprietor: Nanjing University, Nanjing, Jiangsu 210023 (CN)
(72) Inventor: HU, Haidong, Nanjing, Jiangsu 210023 (CN); MA, Sijia, Nanjing, Jiangsu 210023 (CN); REN, Hongqiang, Nanjing, Jiangsu 210023 (CN); XU, Ke, Nanjing, Jiangsu 210023 (CN)
(74) Representative: Niburska, Danuta

(56) References cited:
- WO-A1-2013/010388
- CN-A- 106 145 349
- CN-A- 106 698 856
- CN-A- 108 033 649
- KR-A- 20160 048 501

## Description

This disclosure relates to the field of wastewater treatment, and more particularly to a device and method for pharmaceutical wastewater treatment with high efficiency resource recovery and low energy consumption.

Pharmaceutical wastewater has complex compositions, a wide variety of organic pollutants, high concentration, deep color, high content of solid suspended solids, high chemical oxygen demand (COD) and large fluctuation of influent COD, so it is very difficult to purify. Pharmaceutical wastewater is increasingly threatening the ecological environment and human health. With the strict control of the discharge of pharmaceutical industry and the special discharge standards required by different sensitive areas, local governments have issued a series of policies and plans for energy saving and emission reduction, and pose higher requirements for the effluent from the treatment of pharmaceutical wastewater. Therefore, it is urgent to improve the efficiency, stability and economy of pharmaceutical wastewater treatment process.

At present, the process of "pretreatment + anaerobic treatment + aerobic treatment" is widely used in pharmaceutical wastewater because of its good treatment effect, convenient operation and management, low mud production and certain impact load resistance. The treatment principle is that the pharmaceutical wastewater is first pretreated to remove some refractory pollutants such as oil and particulate matter, and then is subjected to hydrolytic acidification/methane production, so that the organic matter in the influent is mostly removed, reducing the load of the subsequent aerobic treatment, exhibiting a certain impact load resistance. Thereafter, the organic matter and total nitrogen in the anoxic + aerobic section are removed to complete the treatment process of the pharmaceutical wastewater. Hydrolytic acidification can better mitigate the impact of the subsequent treatment stage, but there is often a lower pH value of effluent, affecting the subsequent methane production, and when the hydraulic retention time is short, it is difficult to achieve the desired effect of converting particulate pollutants into dissolved organic matter. Although there is a certain amount of biogas generated in the methanogenesis stage, the gas production per unit volume is low, the quality of the biogas is low, and the formation process of the granular sludge is slow; and the subsequent denitrification process requires the addition of a carbon source to achieve the effluent quality that meets the discharge standard. Under the combined effect of the above problems, it will lead to unstable operation of pharmaceutical wastewater treatment process, low efficiency of carbon resource recovery, and high energy consumption.

Domestic and foreign scholars have done a lot of research on the optimization of pharmaceutical wastewater treatment process, and a variety of physical and chemical methods have been widely used, such as micro-electrolysis, photocatalytic oxidation and membrane separation. However, these processing technologies or processes have problems such as high operating costs, complicated operation management, high requirements on equipment, and low efficiency of resource recovery. For example, Chinese Patent Application No. 201711438146.4 discloses a method for treating pharmaceutical wastewater, which includes evaporating and desalting pharmaceutical wastewater, sequentially introducing the desalted pharmaceutical wastewater to a neutralization tank, an anoxic tank, and an aerobic tank, and finally filtering by using an MBR membrane group. The method does not consider the resource recovery in the wastewater, and the evaporation of the wastewater and filtration through the MBR component increase actual operating costs. Chinese Patent Application No. 201711438146.4 discloses a method for deep treatment of pharmaceutical wastewater, which is subjected to advanced treatment by electrolysis, and the power consumption is high to 40.47 kW·h/m³. These treatment technologies less consider the recycling of resources in pharmaceutical wastewater. The technical solutions adopted are high in energy consumption, complicated in operation and management, and their engineering applicability is poor. Therefore, it is necessary to optimize the pharmaceutical wastewater treatment process. Document WO2013/010388 discloses a multistep method for treating landfill leachate, which, similarly to pharmaceutical wastewater, also contains a wide variety of recalcitrant organic pollutants.

The technical problem solved by the invention is that, in the existing pharmaceutical wastewater treatment process, the pH value of the hydrolyzed acidified effluent is low, which affects the subsequent methane production, and the effect of converting the particulate pollutant into the dissolved organic matter is not satisfactory; and in the methane production process, the production per unit volume is low, the quality of biogas is poor, the formation process of granular sludge is slow, and the subsequent denitrification process requires a large amount of carbon source, resulting in unstable operation of pharmaceutical wastewater treatment process, low efficiency of carbon resource recovery, and current physical chemistry technical means has high energy consumption, complicated operation and management, and poor applicability of the engineering. The disclosure aims to provide a high-efficiency resource recovery and low-energy pharmaceutical wastewater treatment device and its operation method, which can effectively improve the wastewater treatment efficiency, improve the resource recovery rate of the pharmaceutical wastewater treatment, reduce the energy consumption of the treatment process, facilitate the operation and management, and stabilize the treatment process.

To achieve the above objective, provided is a device for pharmaceutical wastewater treatment with high efficiency resource recovery and low energy consumption, the device comprising: a floatation tank; a pH adjusting tank; an acid tank; a base tank; an enhanced hydrolysis acidification tank; a micron calcium silicate tank; a methanogenic tank; an anoxic pool; an aerobic pool; a biogas collection and treatment device; a secondary sedimentation tank; a coagulation sedimentation tank; a polyaluminium chloride (PAC) tank; a control device; and a power supply. The floatation tank, the pH adjusting tank, the enhanced hydrolysis acidification tank, the methanogenic tank, the anoxic pool, the aerobic pool, the secondary sedimentation tank and the coagulation sedimentation tank are connected sequentially; a decontamination device is disposed in the floatation tank; the acid tank and the base tank are independently connected to the pH adjusting tank, and joints thereof are provided with magnetic valves, respectively; the pH adjusting tank is provided with a pH meter, and the pH meter comprises a measuring probe disposed in the pH adjusting tank; the enhanced hydrolysis acidification tank comprises an upper part, a middle part and a lower part; the upper part is connected to the anoxic pool via a draft tube; a fixed filler is disposed in the middle part, and a first uniform water distributor is disposed in the lower part; the first uniform water distributor comprises a main tube and a secondary tube disposed in the main tube; the secondary tube comprises a through hole communicating with a chamber of the main tube; a rotary motor is disposed in the lower part of the enhanced hydrolysis acidification tank to supply power for the main tube; the main tube comprises a main nozzle, and the secondary tube comprises a secondary nozzle; the micron calcium silicate tank is connected to the bottom of the enhanced hydrolysis acidification tank via a guide tube; a second uniform water distributor is disposed in the bottom of the methanogenic tank; a combined filler is disposed in a middle part of the methanogenic tank, and a fixed soft filler is disposed in an upper part of the methanogenic tank; the anoxic pool is filled with a suspension filler; the secondary sedimentation tank is connected to the anoxic pool via a pipe; the PAC tank is connected to the coagulation sedimentation tank; the control device comprises a controller, a processor, and a remote; the controller is connected to the decontamination device, the rotary motor, the pH meter, and the magnetic valves; the processor and the remote are connected to the controller; and the power supply supplies power for the decontamination device, the rotary motor, the pH meter, and the magnetic valves.

As an improvement, the decontamination device comprises decontamination boards, two telescopic rods, a first motor, and a second motor; the two telescopic rods are disposed at two inner ends of the floatation tank, respectively; two sliding blocks are respectively disposed at lower ends of the two telescopic rods; the two sliding blocks are connected via a connecting rod; two ends of the connecting rod are provided with suspension loops, respectively; upper ends of the two telescopic rods are provided with clamping plates, respectively; and two ends of each decontamination board are flexibly connected to the clamping plates via revolving shafts; a slideway is disposed in the bottom wall of the floatation tank, and the two telescopic rods are connected to the floatation tank via the slideway and the two sliding blocks; pulleys are disposed at two bottom ends of the floatation tank; the first motor is disposed at one bottom end of the floatation tank and is provided with a first cable; the first cable winds around one of the pulleys at one bottom end of the floatation tank and connects to one of the suspension loops at one end of the connecting rod; and the second motor is disposed at the other bottom end of the floatation tank 1 and is provided with a second cable; and the second cable winds around the other pulley at the other bottom end of the floatation tank and connects to the other suspension loop at the other end of the connecting rod. The movement of the decontamination boards is pulled by the first motor and the second motor to descale the pollutants on the surface of the floatation tank, and the height of the decontamination board is adjusted by the telescopic rods.

As an improvement, a ball is disposed at the bottom of the sliding blocks, thus reducing the friction between the sliding blocks and the slideway and reducing the energy loss.

As an improvement, the floatation tank is equipped with a liquid level sensor comprising a sensing probe disposed in the floatation tank; the liquid level sensor is connected to the controller. The sensing probe of the liquid level sensor can sense the liquid level in the floatation tank, and transmit the signal to the processor, which sends the instructions to the controller. The controller controls the telescopic rods to adjust the height automatically with the change of liquid level, thus improving the wastewater treatment effect.

As an improvement, five decontamination boards are employed, disposed in parallel, and flexibly clamped by the clamping plates; the clamping plates each are provided with a fixed leg, and a plurality of linear actuators are flexibly disposed between the fixed leg and the five decontamination boards, thus improving the wastewater treatment effect. When the decontamination boards are placed vertically, the pollutant can be scraped off. When the decontamination boards are placed tilted, the treatment effect of the floatation tank will not be affected.

As an improvement, the fixed filler has a specific surface area of 300-410 m²/m³, a density of 1.6-2.1 g/cm³, and a diameter of 150-250 mm, which can intercept the microorganism and suspended particles in the water flow and ensure the stable and efficient hydrolysis and acidification function.

As an improvement, micron calcium silicate in the micron calcium silicate tank has a particle size of 10-100 µm and a density of 2.9-3.1 g/cm³. The calcium silicate particles are absorbed onto the surface of sludge floc and dissociated to yield volatile fatty acids, which enhance the acid production capacity of microorganisms, and calcium ions, which increase the stability of the sludge floc.

As an improvement, the combined filler has a specific surface area of 350-460 m²/m³, a density of 1.2-1.8 g/cm³, and a diameter of 150-250 mm; the fixed soft filler has a density of 1.02-1.1 g/cm³, a fiber bundle length of 60-100 mm, a distance between bundles of 30-45 mm, and a porosity greater than 99%, improving the biogas production capacity and stability of the methanogenic tank.

The disclosure also provides a method for pharmaceutical wastewater treatment with high efficiency resource recovery and low energy consumption using the device, the method comprising:
S1: introducing pharmaceutical wastewater to the floatation tank, removing oil and suspended particles, and eliminating supernatant pollutants of the floatation tank using the decontamination device; allowing the pharmaceutical wastewater to flow into the pH adjusting tank, measuring the pH value of the pharmaceutical wastewater, and opening, by the controller, the magnetic valves of the acid tank or the base tank to adjust the pH value of the pharmaceutical wastewater to be 6.5-8.5;
S2: introducing the pharmaceutical wastewater from the pH adjusting tank to the enhanced hydrolysis acidification tank via the first uniform water distributor, and adding micron calcium silicate to the enhanced hydrolysis acidification tank, so that calcium silicate particles are absorbed onto the surface of sludge floc and dissociated to yield volatile fatty acids, which enhance the acid production capacity of microorganisms, and calcium ions, which increase the stability of the sludge floc, where a mass ratio of the addition amount of the micron calcium silicate to the volatile fatty acids from an effluent of the enhanced hydrolysis acidification tank is 0.06-0.12, and the hydraulic retention time of the pharmaceutical wastewater is 6-8 h, and the fixed filler in the middle part of the enhanced hydrolysis acidification tank is configured to intercept microorganisms and suspended particles in the pharmaceutical wastewater to ensure stable and efficient hydrolysis and acidification;
S3: introducing 5-10% of hydrolytic acidizing fluid from the enhanced hydrolysis acidification tank to the anoxic pool as a carbon source for denitrification, introducing rest of the hydrolytic acidizing fluid to the methanogenic tank, collecting and treating biogas produced in the methanogenic tank by the biogas collection and treatment device, and transporting the biogas as an energy source to sewage treatment plants, where the hydraulic retention time in the methanogenic tank is 15-20 h, the sludge retention time is 20-30 d, calcium ions contained in an effluent of the enhanced hydrolytic acidification tank is beneficial to the formation of granular sludge in the methanogenic tank, methanogenic bacteria is adsorbed on the combined filler in the middle of the methanogenic tank to form a biofilm mainly composed of the methanogenic bacteria; the fixed soft filler in the upper part of the methanogenic tank can intercept the methanogenic bacteria washed out, improving the methane production capacity and stability of the methanogenic tank; and
S4: introducing the wastewater from the methanogenic tank to the anoxic pool, returning an effluent from the secondary sedimentation tank to the anoxic pool, adding a suspension filler having a density of 0.93-0.96 g/cm3, a specific surface area of 400-460 m²/m³, and a diameter of 10-15 mm to the anoxic pool to enrich denitrifying bacteria which adapts to reduce nitrate nitrogen to nitrogen, further introducing the wastewater to the aerobic pool to remove chemical oxygen demand (COD) and ammonia nitrogen, separating the sludge and the water in the secondary sedimentation tank, adding polyaluminium chloride (PAC) to the coagulation sedimentation tank for coagulating sedimentation, to yield final effluent.

Advantages of the device and method for pharmaceutical wastewater treatment with high efficiency resource recovery and low energy consumption of the disclosure are summarized as follows:
(1) The calcium silicate particles are added to the bottom of the enhanced hydrolysis acidification tank and absorbed onto the surface of sludge floc, which can effectively increase the stability of the sludge flocs, and the volatile fatty acids produced by microorganisms can be rapidly dissociated on the surface of sludge flocs, thus reducing the adverse effects of pH drop caused by the accumulation of the volatile fatty acids on the microorganisms, improving the acid production ability of the sludge, and ensuring the pH value of the effluent does not decrease to such a value that poses a negative effect on the methanogenic stage. The combined filler in the middle of the tank can effectively ensure that the microorganisms are not washed out of the tank and can intercept part of the suspended solids.
(2) The disclosure adopts an efficient methanogenic tank. First, the calcium ions contained in the effluent of the hydrolytic acidification tank facilitate the formation of granular sludge in the methanogenic tank. The combined filler and the soft filler are respectively arranged in the middle and upper parts of the methanogenic tank. Methanogenic bacteria can be adsorbed on the fixed combined filler in the middle of the methanogenic tank to form a biofilm dominated by the methanogenic bacteria, thus greatly improving the anti-impact load capacity. The fixed soft filler on the upper part of the methanogenic tank can intercept the methanogenic bacteria washed out, further improving the methanogenic capacity of the methanogenic tank, and achieving the efficient recovery of carbon resources.
(3) The disclosure adopts the effluent of the enhanced hydrolysis acidification tank as an additional carbon source for denitrification in the anoxic tank, greatly reducing the dosage of the additional carbon source, and adds a suspended filler to the anoxic tank for enriching the denitrifying bacteria, thus improving the anti-impact load capacity of the anoxic tank. The calcium silicate required in the disclosure is cheap and easy to obtain. The device has simple structure and convenient operation. The method can effectively improve the efficiency and stability of the wastewater treatment. The recovery rate of the carbon resources is high and the energy consumption is low. The treatment process of the pharmaceutical wastewater is stable, efficient and energy-saving.
   FIG. 1 is a schematic diagram of a device for pharmaceutical wastewater treatment of the disclosure;
   FIG. 2 is a schematic diagram of a floatation tank of the disclosure;
   FIG. 3 is a schematic diagram of a decontamination device of the disclosure;
   FIG. 4 is a side view of a decontamination device comprising vertically disposed decontamination boards of the disclosure;
   FIG. 5 is a side view of a decontamination device comprising slantwise disposed decontamination boards of the disclosure;
   FIG. 6 is a schematic diagram of a uniform water distributor of the disclosure; and
   FIG. 7 is a top view of a uniform water distributor of the disclosure.

In the drawings, the following reference numbers are used: 1. Floatation tank; 101. Liquid level sensor; 2. pH adjusting tank; 3. Acid tank; 4. Base tank; 5. Enhanced hydrolysis acidification tank; 6. Micron calcium silicate tank; 7. Fixed filler; 8. Methanogenic tank; 9. Combined filler; 10. Fixed soft filler; 11. Anoxic pool; 12. Aerobic pool; 13. Biogas collection and treatment device; 14. Secondary sedimentation tank; 15. Coagulation sedimentation tank; 16. Polyaluminium chloride (PAC) tank; 17. Decontamination device; 170. Decontamination board; 171. Telescopic rod; 172. First motor; 173. Second motor; 174. Sliding block; 175. Clamping plate; 176. Slideway; 177. Pulley; 178. Fixed leg; 179. Linear actuator; 18. Uniform water Distributor; 180. Main tube; 181. Secondary tube; 182. Rotary motor; 183. Main nozzle; 184. Secondary nozzle; 19. Control device; 190. Controller; 191. Processor; 192. Remote; 21. pH meter.

### Example 1

In this example, a small test device was used to treat the pharmaceutical wastewater from the workshop of a fermentation pharmaceutical enterprise. The wastewater presents orange color, with a wide range of organics. The water intake is 30 L/d, COD 14500-17000 mg/L, pH 6.2-6.9, and turbidity 179-226 mg/L.

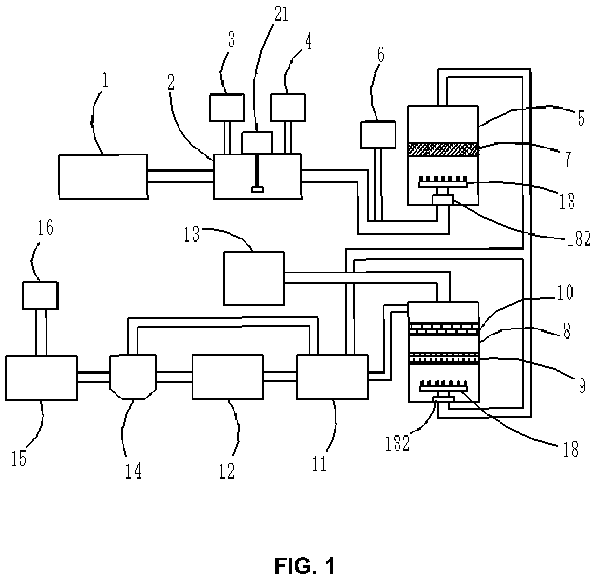

The small test device is shown in FIG. 1, which is a pharmaceutical wastewater treatment device with high efficiency resource recovery and low energy consumption, and comprises a floatation tank 1; a pH adjusting tank 2; an acid tank 3; a base tank 4; an enhanced hydrolysis acidification tank 5; a micron calcium silicate tank 6; a methanogenic tank 8; an anoxic pool 11; an aerobic pool 12; a biogas collection and treatment device 13; a secondary sedimentation tank 14; a coagulation sedimentation tank 15; a polyaluminium chloride (PAC) tank 16; a control device 19; and a power supply. The floatation tank 1, the pH adjusting tank 2, the enhanced hydrolysis acidification tank 5, the methanogenic tank 8, the anoxic pool 11, the aerobic pool 12, the secondary sedimentation tank 14 and the coagulation sedimentation tank 15 are connected sequentially.

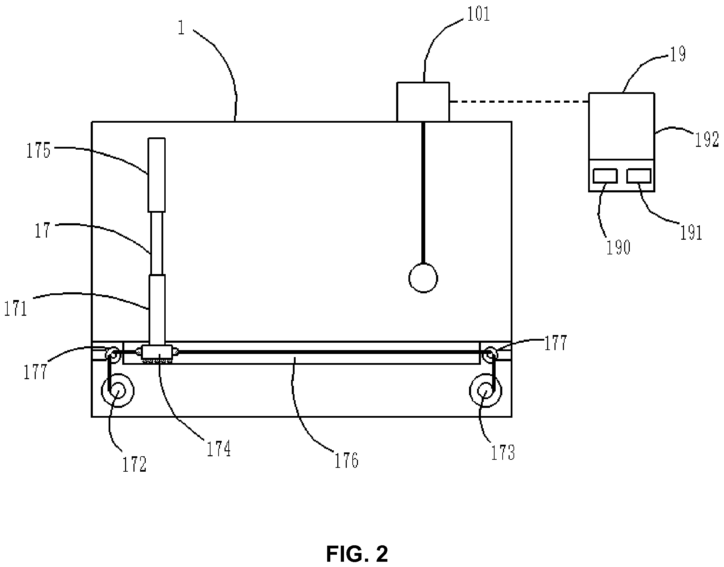

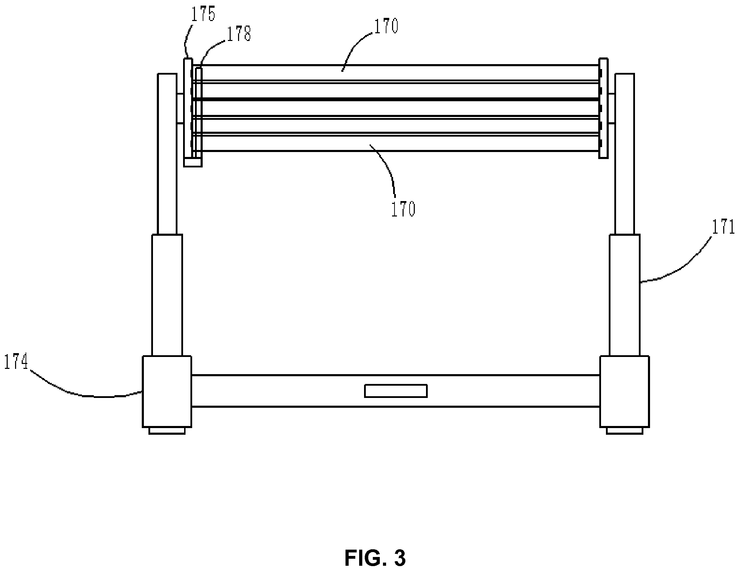

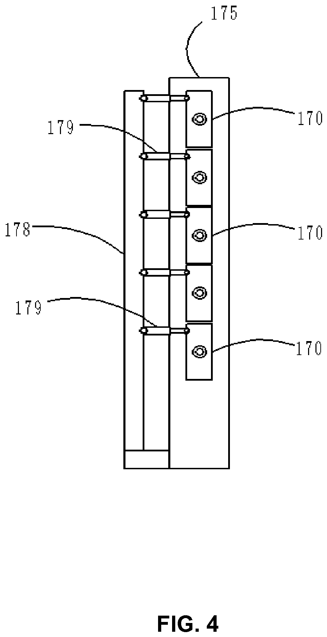

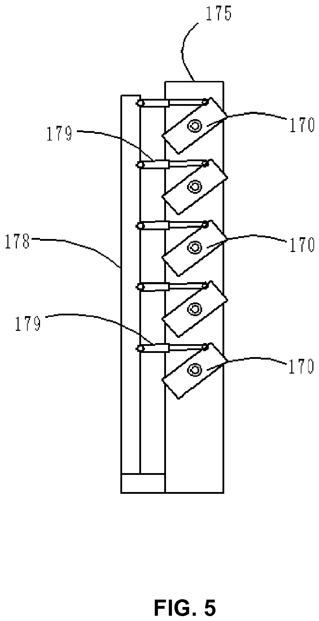

As shown in FIGS. 2, 3, 4 and 5, the floatation tank 1 is equipped with a liquid level sensor 101 comprising a sensing probe disposed in the floatation tank 1. The liquid level sensor 101 is connected to the controller. A decontamination device 17 is disposed in the floatation tank 1. The decontamination device 17 comprises decontamination boards 170, two telescopic rods 171, a first motor 172, and a second motor 173; the two telescopic rods 171 are disposed at two inner ends of the floatation tank 1, respectively; two sliding blocks 174 are respectively disposed at lower ends of the two telescopic rods 171; the two sliding blocks 174 are connected via a connecting rod; two ends of the connecting rod are provided with suspension loops, respectively; the upper ends of the two telescopic rods 171 are provided with clamping plates 175, respectively. Five decontamination boards 170 are employed, disposed in parallel, and flexibly clamped by the clamping plates 175; the clamping plates 175 each are provided with a fixed leg 178, and a plurality of linear actuators 179 are flexibly disposed between the fixed leg 178 and the five decontamination boards 170. A slideway 176 is disposed in the bottom wall of the floatation tank 1, and the two telescopic rods 171 are connected to the floatation tank 1 via the slideway 176 and the two sliding blocks 174; pulleys 177 are disposed at two bottom ends of the floatation tank 1; the first motor 172 is disposed at one bottom end of the floatation tank 1 and is provided with a first cable; the first cable winds around one of the pulleys 177 at one bottom end of the floatation tank 1 and connects to one of the suspension loops at one end of the connecting rod; and the second motor 173 is disposed at the other bottom end of the floatation tank 1 and is provided with a second cable; and the second cable winds around the other pulley 177 at the other bottom end of the floatation tank 1 and connects to the other suspension loop at the other end of the connecting rod. A ball is disposed at the bottom of the sliding blocks 174.

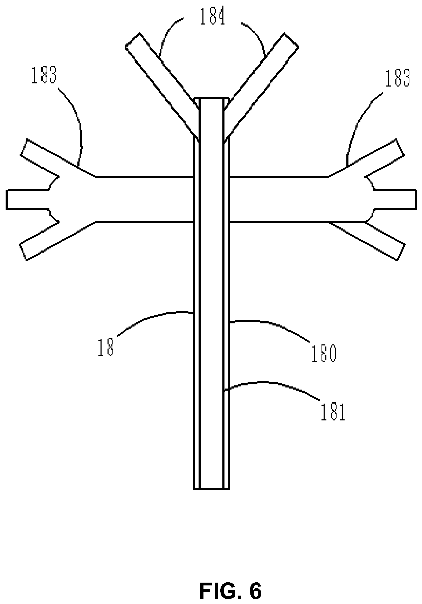

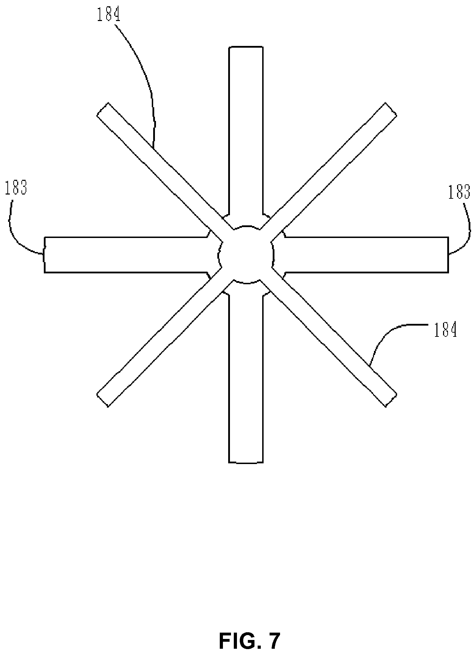

As shown in FIGS. 6 and 7, the acid tank 3 and the base tank 4 are independently connected to the pH adjusting tank 2, and joints thereof are provided with magnetic valves, respectively; the pH adjusting tank 2 is provided with a pH meter 21, and the pH meter 21 comprises a measuring probe disposed in the pH adjusting tank 2; the enhanced hydrolysis acidification tank 5 comprises an upper part, a middle part and a lower part; the upper part is connected to the anoxic pool 11 via a draft tube; a fixed filler 7 is disposed in the middle part, and has a specific surface area of 300 m²/m³, a density of 2.1 g/cm³, and a diameter of 150 mm. A first uniform water distributor 18 is disposed in the lower part of the enhanced hydrolysis acidification tank 5; the first uniform water distributor 18 comprises a main tube 180 and a secondary tube 181 disposed in the main tube 180; the secondary tube 181 comprises a through hole communicating with a chamber of the main tube 180; a rotary motor 182 is disposed in the lower part of the enhanced hydrolysis acidification tank 5 to supply power for the main tube 180; the main tube 180 comprises a main nozzle 183, and the secondary tube 181 comprises a secondary nozzle 184; the micron calcium silicate tank 6 is connected to the bottom of the enhanced hydrolysis acidification tank 5 via a guide tube; the micron calcium silicate in the micron calcium silicate tank has a particle size of 10 µm and a density of 2.9 g/cm³.

As shown in FIG. 1, a second uniform water distributor 18 is disposed in the bottom of the methanogenic tank 8; a combined filler 9 is disposed in a middle part of the methanogenic tank 8, and a fixed soft filler 10 is disposed in an upper part of the methanogenic tank 8; the combined filler 9 has a specific surface area of 350 m²/m³, a density of 1.8 g/cm³, and a diameter of 150 mm; the fixed soft filler 10 has a density of 1.1 g/cm³, a fiber bundle length of 100 mm, a distance between bundles of 45 mm, and a porosity greater than 99%. The anoxic pool 11 is filled with a suspension filler; the secondary sedimentation tank 14 is connected to the anoxic pool 11 via a pipe; the PAC tank 16 is connected to the coagulation sedimentation tank 15; the control device 19 comprises a controller 190, a processor 191, and a remote 192; the controller 190 is commercially available and is connected to the decontamination device 17, the rotary motor 182, the pH meter 21, and the magnetic valves; the processor 191 and the remote 192 are connected to the controller 190; and the power supply supplies power for the decontamination device 17, the rotary motor 182, the pH meter 21, and the magnetic valves.

A method for pharmaceutical wastewater treatment with high efficiency resource recovery and low energy consumption using the aforesaid device comprises the following steps:
S1: introducing pharmaceutical wastewater to the floatation tank 1, removing oil and suspended particles, and eliminating supernatant pollutants of the floatation tank 1 using the decontamination device 17; allowing the pharmaceutical wastewater to flow into the pH adjusting tank 2, measuring a pH value of the pharmaceutical wastewater, and opening, by the controller 190, the magnetic valves of the acid tank 3 or the base tank 4 to adjust the pH value of the pharmaceutical wastewater to be 6.5;
S2: introducing the pharmaceutical wastewater from the pH adjusting tank 2 to the enhanced hydrolysis acidification tank 5 via the first uniform water distributor 18, and adding micron calcium silicate to the enhanced hydrolysis acidification tank 5, so that calcium silicate particles are absorbed onto the surface of sludge floc and dissociated to yield volatile fatty acids, which enhance the acid production capacity of microorganisms, and calcium ions, which increase the stability of the sludge floc, where a mass ratio of the addition amount of the micron calcium silicate to the volatile fatty acids from an effluent of the enhanced hydrolysis acidification tank 5 is 0.06, and the hydraulic retention time of the pharmaceutical wastewater is 6 h, and the fixed filler 7 in the middle part of the enhanced hydrolysis acidification tank is configured to intercept microorganisms and suspended particles in the pharmaceutical wastewater to ensure stable and efficient hydrolysis and acidification;
S3: introducing 6% of hydrolytic acidizing fluid from the enhanced hydrolysis acidification tank 5 to the anoxic pool 11 as a carbon source for denitrification, introducing rest of the hydrolytic acidizing fluid to the methanogenic tank 8, collecting and treating biogas produced in the methanogenic tank by the biogas collection and treatment device 13, and transporting the biogas as an energy source to sewage treatment plants, where the hydraulic retention time in the methanogenic tank 8 is 15 h, the sludge retention time is 20 d, operation for 90 days, calcium ions contained in an effluent of the enhanced hydrolytic acidification tank 5 is beneficial to the formation of granular sludge in the methanogenic tank, methanogenic bacteria is adsorbed on the combined filler 9 in the middle of the methanogenic tank 8 to form a biofilm mainly composed of the methanogenic bacteria; the fixed soft filler 10 in the upper part of the methanogenic tank can intercept the methanogenic bacteria washed out, improving the methane production capacity and stability of the methanogenic tank 8; and
S4: introducing the wastewater from the methanogenic tank 8 to the anoxic pool 11, returning an effluent from the secondary sedimentation tank 14 to the anoxic pool 11, adding a suspension filler having a density of 0.93 g/cm³, a specific surface area of 400 m²/m³, and a diameter of 10 mm to the anoxic pool 11 to enrich denitrifying bacteria which adapts to reduce nitrate nitrogen to nitrogen, further introducing the wastewater to the aerobic pool 12 to remove chemical oxygen demand (COD) and ammonia nitrogen, where the COD removal rate of the two-phase anaerobic treatment process is maintained at 82.3%-86.7%, the effluent COD is 2154-2932 mg/L, and the methane yield of the methanogenic tank is 261-296 mL/g COD; separating the sludge and the water in the secondary sedimentation tank 14, adding polyaluminium chloride (PAC) to the coagulation sedimentation tank 15 for coagulating sedimentation, to yield final effluent, where the effluent COD is 167-342 mg/L, the total nitrogen (TN) concentration is 18-31 mg/L, suspended solids (SS) < 31 mg/L, and the color is 5-9.

### Example 2

Different from that in Example 1, the treated pharmaceutical wastewater is a mixed effluent from a workshop of a pharmaceutical enterprise. The wastewater presents orange red, with a wide range of organics. The water intake is 50 L/d, COD 12000-13900 mg/L, pH 7-7.2, and turbidity 148-169 mg/L.

The small test device is shown in FIG. 1, which is a pharmaceutical wastewater treatment device with high efficiency resource recovery and low energy consumption, and comprises a floatation tank 1; a pH adjusting tank 2; an acid tank 3; a base tank 4; an enhanced hydrolysis acidification tank 5; a micron calcium silicate tank 6; a methanogenic tank 8; an anoxic pool 11; an aerobic pool 12; a biogas collection and treatment device 13; a secondary sedimentation tank 14; a coagulation sedimentation tank 15; a polyaluminium chloride (PAC) tank 16; a control device 19; and a power supply. The floatation tank 1, the pH adjusting tank 2, the enhanced hydrolysis acidification tank 5, the methanogenic tank 8, the anoxic pool 11, the aerobic pool 12, the secondary sedimentation tank 14 and the coagulation sedimentation tank 15 are connected sequentially.

As shown in FIGS. 2, 3, 4 and 5, the floatation tank 1 is equipped with a liquid level sensor 101 comprising a sensing probe disposed in the floatation tank 1. The liquid level sensor 101 is connected to the controller. A decontamination device 17 is disposed in the floatation tank 1. The decontamination device 17 comprises decontamination boards 170, two telescopic rods 171, a first motor 172, and a second motor 173; the two telescopic rods 171 are disposed at two inner ends of the floatation tank 1, respectively; two sliding blocks 174 are respectively disposed at lower ends of the two telescopic rods 171; the two sliding blocks 174 are connected via a connecting rod; two ends of the connecting rod are provided with suspension loops, respectively; the upper ends of the two telescopic rods 171 are provided with clamping plates 175, respectively. Five decontamination boards 170 are employed, disposed in parallel, and flexibly clamped by the clamping plates 175; the clamping plates 175 each are provided with a fixed leg 178, and a plurality of linear actuators 179 are flexibly disposed between the fixed leg 178 and the five decontamination boards 170. A slideway 176 is disposed in the bottom wall of the floatation tank 1, and the two telescopic rods 171 are connected to the floatation tank 1 via the slideway 176 and the two sliding blocks 174; pulleys 177 are disposed at two bottom ends of the floatation tank 1; the first motor 172 is disposed at one bottom end of the floatation tank 1 and is provided with a first cable; the first cable winds around one of the pulleys 177 at one bottom end of the floatation tank 1 and connects to one of the suspension loops at one end of the connecting rod; and the second motor 173 is disposed at the other bottom end of the floatation tank 1 and is provided with a second cable; and the second cable winds around the other pulley 177 at the other bottom end of the floatation tank 1 and connects to the other suspension loop at the other end of the connecting rod. A ball is disposed at the bottom of the sliding blocks 174.

As shown in FIGS. 6 and 7, the acid tank 3 and the base tank 4 are independently connected to the pH adjusting tank 2, and joints thereof are provided with magnetic valves, respectively; the pH adjusting tank 2 is provided with a pH meter 21, and the pH meter 21 comprises a measuring probe disposed in the pH adjusting tank 2; the enhanced hydrolysis acidification tank 5 comprises an upper part, a middle part and a lower part; the upper part is connected to the anoxic pool 11 via a draft tube; a fixed filler 7 is disposed in the middle part, and has a specific surface area of 350 m²/m³, a density of 2 g/cm³, and a diameter of 200 mm. A first uniform water distributor 18 is disposed in the lower part of the enhanced hydrolysis acidification tank 5; the first uniform water distributor 18 comprises a main tube 180 and a secondary tube 181 disposed in the main tube 180; the secondary tube 181 comprises a through hole communicating with a chamber of the main tube 180; a rotary motor 182 is disposed in the lower part of the enhanced hydrolysis acidification tank 5 to supply power for the main tube 180; the main tube 180 comprises a main nozzle 183, and the secondary tube 181 comprises a secondary nozzle 184; the micron calcium silicate tank 6 is connected to the bottom of the enhanced hydrolysis acidification tank 5 via a guide tube; the micron calcium silicate in the micron calcium silicate tank has a particle size of 50 µm and a density of 3 g/cm³.

As shown in FIG. 1, a second uniform water distributor 18 is disposed in the bottom of the methanogenic tank 8; a combined filler 9 is disposed in a middle part of the methanogenic tank 8, and a fixed soft filler 10 is disposed in an upper part of the methanogenic tank 8; the combined filler 9 has a specific surface area of 380 m²/m³, a density of 1.7 g/cm³, and a diameter of 200 mm; the fixed soft filler 10 has a density of 1.05 g/cm³, a fiber bundle length of 80 mm, a distance between bundles of 35 mm, and a porosity greater than 99%. The anoxic pool 11 is filled with a suspension filler; the secondary sedimentation tank 14 is connected to the anoxic pool 11 via a pipe; the PAC tank 16 is connected to the coagulation sedimentation tank 15; the control device 19 comprises a controller 190, a processor 191, and a remote 192; the controller 190 is commercially available and is connected to the decontamination device 17, the rotary motor 182, the pH meter 21, and the magnetic valves; the processor 191 and the remote 192 are connected to the controller 190; and the power supply supplies power for the decontamination device 17, the rotary motor 182, the pH meter 21, and the magnetic valves.

A method for pharmaceutical wastewater treatment with high efficiency resource recovery and low energy consumption using the aforesaid device comprises the following steps:
S1: introducing pharmaceutical wastewater to the floatation tank 1, removing oil and suspended particles, and eliminating supernatant pollutants of the floatation tank 1 using the decontamination device 17; allowing the pharmaceutical wastewater to flow into the pH adjusting tank 2, measuring a pH value of the pharmaceutical wastewater, and opening, by the controller 190, the magnetic valves of the acid tank 3 or the base tank 4 to adjust the pH value of the pharmaceutical wastewater to be 6.5;
S2: introducing the pharmaceutical wastewater from the pH adjusting tank 2 to the enhanced hydrolysis acidification tank 5 via the first uniform water distributor 18, and adding micron calcium silicate to the enhanced hydrolysis acidification tank 5, so that calcium silicate particles are absorbed onto the surface of sludge floc and dissociated to yield volatile fatty acids, which enhance the acid production capacity of microorganisms, and calcium ions, which increase the stability of the sludge floc, where a mass ratio of the addition amount of the micron calcium silicate to the volatile fatty acids from an effluent of the enhanced hydrolysis acidification tank 5 is 0.09, and the hydraulic retention time of the pharmaceutical wastewater is 7 h, and the fixed filler 7 in the middle part of the enhanced hydrolysis acidification tank is configured to intercept microorganisms and suspended particles in the pharmaceutical wastewater to ensure stable and efficient hydrolysis and acidification;
S3: introducing 7% of hydrolytic acidizing fluid from the enhanced hydrolysis acidification tank 5 to the anoxic pool 11 as a carbon source for denitrification, introducing rest of the hydrolytic acidizing fluid to the methanogenic tank 8, collecting and treating biogas produced in the methanogenic tank by the biogas collection and treatment device 13, and transporting the biogas as an energy source to sewage treatment plants, where the hydraulic retention time in the methanogenic tank 8 is 15 h, a sludge retention time is 25 d, operation for 70 days, calcium ions contained in an effluent of the enhanced hydrolytic acidification tank 5 is beneficial to the formation of granular sludge in the methanogenic tank, methanogenic bacteria is adsorbed on the combined filler 9 in the middle of the methanogenic tank 8 to form a biofilm mainly composed of the methanogenic bacteria; the fixed soft filler 10 in the upper part of the methanogenic tank can intercept the methanogenic bacteria washed out, improving the methane production capacity and stability of the methanogenic tank 8; and
S4: introducing the wastewater from the methanogenic tank 8 to the anoxic pool 11, returning an effluent from the secondary sedimentation tank 14 to the anoxic pool 11, adding a suspension filler having a density of 0.95 g/cm³, a specific surface area of 420 m²/m³, and a diameter of 12 mm to the anoxic pool 11 to enrich denitrifying bacteria which adapts to reduce nitrate nitrogen to nitrogen, further introducing the wastewater to the aerobic pool 12 to remove chemical oxygen demand (COD) and ammonia nitrogen, where the COD removal rate of the two-phase anaerobic treatment process is maintained at 81.1 %-84.3%, the effluent COD is 1884-2627 mg/L, and the methane yield of the methanogenic tank is 268-304 mL/g COD; separating the sludge and the water in the secondary sedimentation tank 14, adding polyaluminium chloride (PAC) to the coagulation sedimentation tank 15 for coagulating sedimentation, to yield final effluent, where the effluent COD is 203-412 mg/L, the total nitrogen (TN) concentration is 26-43 mg/L, suspended solids (SS) < 37 mg/L, and the color is 4-11.

### Example 3

In this example, a pilot test device is used to treat the pharmaceutical wastewater, which was the fermentation wastewater of an anti-cancer drug, with COD of 18500-21900 mg /L, pH of 6.4-6.7, suspended solids (SS) of 764-1128 mg /L, and the water intake is 4 m³/d.

The pilot test device is shown in FIG. 1, which is a pharmaceutical wastewater treatment device with high efficiency resource recovery and low energy consumption, and comprises a floatation tank 1; a pH adjusting tank 2; an acid tank 3; a base tank 4; an enhanced hydrolysis acidification tank 5; a micron calcium silicate tank 6; a methanogenic tank 8; an anoxic pool 11; an aerobic pool 12; a biogas collection and treatment device 13; a secondary sedimentation tank 14; a coagulation sedimentation tank 15; a polyaluminium chloride (PAC) tank 16; a control device 19; and a power supply. The floatation tank 1, the pH adjusting tank 2, the enhanced hydrolysis acidification tank 5, the methanogenic tank 8, the anoxic pool 11, the aerobic pool 12, the secondary sedimentation tank 14 and the coagulation sedimentation tank 15 are connected sequentially.

As shown in FIGS. 2, 3, 4 and 5, the floatation tank 1 is equipped with a liquid level sensor 101 comprising a sensing probe disposed in the floatation tank 1. The liquid level sensor 101 is connected to the controller. A decontamination device 17 is disposed in the floatation tank 1. The decontamination device 17 comprises decontamination boards 170, two telescopic rods 171, a first motor 172, and a second motor 173; the two telescopic rods 171 are disposed at two inner ends of the floatation tank 1, respectively; two sliding blocks 174 are respectively disposed at lower ends of the two telescopic rods 171; the two sliding blocks 174 are connected via a connecting rod; two ends of the connecting rod are provided with suspension loops, respectively; the upper ends of the two telescopic rods 171 are provided with clamping plates 175, respectively. Five decontamination boards 170 are employed, disposed in parallel, and flexibly clamped by the clamping plates 175; the clamping plates 175 each are provided with a fixed leg 178, and a plurality of linear actuators 179 are flexibly disposed between the fixed leg 178 and the five decontamination boards 170. A slideway 176 is disposed in the bottom wall of the floatation tank 1, and the two telescopic rods 171 are connected to the floatation tank 1 via the slideway 176 and the two sliding blocks 174; pulleys 177 are disposed at two bottom ends of the floatation tank 1; the first motor 172 is disposed at one bottom end of the floatation tank 1 and is provided with a first cable; the first cable winds around one of the pulleys 177 at one bottom end of the floatation tank 1 and connects to one of the suspension loops at one end of the connecting rod; and the second motor 173 is disposed at the other bottom end of the floatation tank 1 and is provided with a second cable; and the second cable winds around the other pulley 177 at the other bottom end of the floatation tank 1 and connects to the other suspension loop at the other end of the connecting rod. A ball is disposed at the bottom of the sliding blocks 174.

As shown in FIGS. 6 and 7, the acid tank 3 and the base tank 4 are independently connected to the pH adjusting tank 2, and joints thereof are provided with magnetic valves, respectively; the pH adjusting tank 2 is provided with a pH meter 21, and the pH meter 21 comprises a measuring probe disposed in the pH adjusting tank 2; the enhanced hydrolysis acidification tank 5 comprises an upper part, a middle part and a lower part; the upper part is connected to the anoxic pool 11 via a draft tube; a fixed filler 7 is disposed in the middle part, and has a specific surface area of 410 m²/m³, a density of 1.6 g/cm³, and a diameter of 250 mm. A first uniform water distributor 18 is disposed in the lower part of the enhanced hydrolysis acidification tank 5; the first uniform water distributor 18 comprises a main tube 180 and a secondary tube 181 disposed in the main tube 180; the secondary tube 181 comprises a through hole communicating with a chamber of the main tube 180; a rotary motor 182 is disposed in the lower part of the enhanced hydrolysis acidification tank 5 to supply power for the main tube 180; the main tube 180 comprises a main nozzle 183, and the secondary tube 181 comprises a secondary nozzle 184; the micron calcium silicate tank 6 is connected to the bottom of the enhanced hydrolysis acidification tank 5 via a guide tube; the micron calcium silicate in the micron calcium silicate tank has a particle size of 80 µm and a density of 2.9 g/cm³.

As shown in FIG. 1, a second uniform water distributor 18 is disposed in the bottom of the methanogenic tank 8; a combined filler 9 is disposed in a middle part of the methanogenic tank 8, and a fixed soft filler 10 is disposed in an upper part of the methanogenic tank 8; the combined filler 9 has a specific surface area of 460 m²/m³, a density of 1.2 g/cm³, and a diameter of 250 mm; the fixed soft filler 10 has a density of 1.02 g/cm³, a fiber bundle length of 100 mm, a distance between bundles of 30 mm, and a porosity greater than 99%. The anoxic pool 11 is filled with a suspension filler; the secondary sedimentation tank 14 is connected to the anoxic pool 11 via a pipe; the PAC tank 16 is connected to the coagulation sedimentation tank 15; the control device 19 comprises a controller 190, a processor 191, and a remote 192; the controller 190 is commercially available and is connected to the decontamination device 17, the rotary motor 182, the pH meter 21, and the magnetic valves; the processor 191 and the remote 192 are connected to the controller 190; and the power supply supplies power for the decontamination device 17, the rotary motor 182, the pH meter 21, and the magnetic valves.

A method for pharmaceutical wastewater treatment with high efficiency resource recovery and low energy consumption using the aforesaid device comprises the following steps:
S1: introducing pharmaceutical wastewater to the floatation tank 1, removing oil and suspended particles, and eliminating supernatant pollutants of the floatation tank 1 using the decontamination device 17; allowing the pharmaceutical wastewater to flow into the pH adjusting tank 2, measuring a pH value of the pharmaceutical wastewater, and opening, by the controller 190, the magnetic valves of the acid tank 3 or the base tank 4 to adjust the pH value of the pharmaceutical wastewater to be 7.5, followed by adding a base to increase the pH of the wastewater to be greater than 7.5;
S2: introducing the pharmaceutical wastewater from the pH adjusting tank 2 to the enhanced hydrolysis acidification tank 5 via the first uniform water distributor 18, and adding micron calcium silicate to the enhanced hydrolysis acidification tank 5, so that calcium silicate particles are absorbed onto the surface of sludge floc and dissociated to yield volatile fatty acids, which enhance the acid production capacity of microorganisms, and calcium ions, which increase the stability of the sludge floc, where a mass ratio of the addition amount of the micron calcium silicate to the volatile fatty acids from an effluent of the enhanced hydrolysis acidification tank 5 is 0.12, and the hydraulic retention time of the pharmaceutical wastewater is 8 h, and the fixed filler 7 in the middle part of the enhanced hydrolysis acidification tank is configured to intercept microorganisms and suspended particles in the pharmaceutical wastewater to ensure stable and efficient hydrolysis and acidification;
S3: introducing 8% of hydrolytic acidizing fluid from the enhanced hydrolysis acidification tank 5 to the anoxic pool 11 as a carbon source for denitrification, introducing rest of the hydrolytic acidizing fluid to the methanogenic tank 8, collecting and treating biogas produced in the methanogenic tank by the biogas collection and treatment device 13, and transporting the biogas as an energy source to sewage treatment plants, where the hydraulic retention time in the methanogenic tank 8 is 20 h, a sludge retention time is 20 d, operation for 155 days, calcium ions contained in an effluent of the enhanced hydrolytic acidification tank 5 is beneficial to the formation of granular sludge in the methanogenic tank, methanogenic bacteria is adsorbed on the combined filler 9 in the middle of the methanogenic tank 8 to form a biofilm mainly composed of the methanogenic bacteria; the fixed soft filler 10 in the upper part of the methanogenic tank can intercept the methanogenic bacteria washed out, improving the methane production capacity and stability of the methanogenic tank 8; and
S4: introducing the wastewater from the methanogenic tank 8 to the anoxic pool 11, returning an effluent from the secondary sedimentation tank 14 to the anoxic pool 11, adding a suspension filler having a density of 0.96 g/cm³, a specific surface area of 460 m²/m³, and a diameter of 15 mm to the anoxic pool 11 to enrich denitrifying bacteria which adapts to reduce nitrate nitrogen to nitrogen, further introducing the wastewater to the aerobic pool 12 to remove chemical oxygen demand (COD) and ammonia nitrogen, where the COD removal rate of the two-phase anaerobic treatment process is maintained at 76.7%-84.2%, the effluent COD is 2981-4629 mg/L, the hydraulic retention time of the wastewater in the anoxic pool 11 and the aerobic pool 12 in this example is 1.5 times of the above two examples respectively, and the methane yield of the methanogenic tank is 279-318 mL/g COD; separating the sludge and the water in the secondary sedimentation tank 14, adding polyaluminium chloride (PAC) to the coagulation sedimentation tank 15 for coagulating sedimentation, to yield final effluent, where the effluent COD is 327-482 mg/L, the total nitrogen (TN) concentration is 34-53 mg/L, suspended solids (SS) < 42 mg/L, and the color is 16-31.

It will be obvious to those skilled in the art that changes and modifications may be made within the scope of the invention that is determined by the claims.

## Claims

1. A device for pharmaceutical wastewater treatment with high efficiency resource recovery and low energy consumption, **characterized by** comprising:
a floatation tank (1);
a pH adjusting tank (2);
an acid tank (3);
a base tank (4);
an enhanced hydrolysis acidification tank (5);
a micron calcium silicate tank (6);
a methanogenic tank (8);
an anoxic pool (11);
an aerobic pool (12);
a biogas collection and treatment device (13);
a secondary sedimentation tank (14);
a coagulation sedimentation tank (15);
a polyaluminium chloride (PAC) tank (16);
a control device (19); and
a power supply;
wherein:
the floatation tank (1), the pH adjusting tank (2), the enhanced hydrolysis acidification tank (5), the methanogenic tank (8), the anoxic pool (11), the aerobic pool (12), the secondary sedimentation tank (14) and the coagulation sedimentation tank (15) are connected sequentially;
a decontamination device (17) allowing to eliminate supernatant pollutants of the flotation tank is disposed in the floatation tank (1);
the acid tank (3) and the base tank (4) are independently connected to the pH adjusting tank (2), and joints thereof are provided with magnetic valves, respectively;
the pH adjusting tank (2) is provided with a pH meter (21), and the pH meter (21) comprises a measuring probe disposed in the pH adjusting tank (2);
the enhanced hydrolysis acidification tank (5) comprises an upper part, a middle part and a lower part; the upper part is connected to the anoxic pool (11) via a draft tube; a fixed filler (7) is disposed in the middle part, and a first uniform water distributor (18) is disposed in the lower part;
the first uniform water distributor (18) comprises a main tube (180) and a secondary tube (181) disposed in the main tube (180); the secondary tube (181) comprises a through hole communicating with a chamber of the main tube (180);
a rotary motor (182) is disposed in the lower part of the enhanced hydrolysis acidification tank (5) to supply power for the main tube (180); the main tube (180) comprises a main nozzle (183), and the secondary tube (181) comprises a secondary nozzle (184);
the micron calcium silicate tank (6) is connected to a bottom of the enhanced hydrolysis acidification tank (5) via a guide tube;
a second uniform water distributor (18) is disposed in a bottom of the methanogenic tank (8); a combined filler (9) is disposed in a middle part of the methanogenic tank (8), and a fixed soft filler (10) is disposed in an upper part of the methanogenic tank (8);
the anoxic pool (11) is filled with a suspension filler;
the secondary sedimentation tank (14) is connected to the anoxic pool (11) via a pipe;
the PAC tank (16) is connected to the coagulation sedimentation tank (15);
the control device (19) comprises a controller (190), a processor (191), and a remote (192);
the controller (190) is connected to the decontamination device (17), the rotary motor (182), the pH meter (21), and the magnetic valves;
the processor (191) and the remote (192) are connected to the controller (190); and
the power supply supplies power for the decontamination device (17), the rotary motor (182), the pH meter (21), and the magnetic valves.

2. The device of claim 1, **characterized in that:**
the decontamination device (17) comprises decontamination boards (170), two telescopic rods (171), a first motor (172), and a second motor (173);
the two telescopic rods (171) are disposed at two inner ends of the floatation tank (1), respectively;
two sliding blocks (174) are respectively disposed at lower ends of the two telescopic rods (171);
the two sliding blocks (174) are connected via a connecting rod; two ends of the connecting rod are provided with suspension loops, respectively;
upper ends of the two telescopic rods (171) are provided with clamping plates (175), respectively; and two ends of each decontamination board (170) are flexibly connected to the clamping plates (175) via revolving shafts;
a slideway (176) is disposed in a bottom wall of the floatation tank (1), and the two telescopic rods (171) are connected to the floatation tank (1) via the slideway (176) and the two sliding blocks (174);
pulleys (177) are disposed at two bottom ends of the floatation tank (1); the first motor (172) is disposed at one bottom end of the floatation tank (1) and is provided with a first cable; the first cable winds around one of the pulleys (177) at one bottom end of the floatation tank (1) and connects to one of the suspension loops at one end of the connecting rod; and
the second motor (173) is disposed at the other bottom end of the floatation tank (1) and is provided with a second cable; and the second cable winds around the other pulley (177) at the other bottom end of the floatation tank (1) and connects to the other suspension loop at the other end of the connecting rod.

3. The device of claim 2, **characterized in that** a ball is disposed at a bottom of the sliding blocks (174).

4. The device of claim 2, **characterized in that** five decontamination boards (170) are employed, disposed in parallel, and flexibly clamped by the clamping plates (175); the clamping plates (175) each are provided with a fixed leg (178), and a plurality of linear actuators (179) are flexibly disposed between the fixed leg (178) and the five decontamination boards (170).

5. The device of claim 1, **characterized in that** the fixed filler (7) has a specific surface area of 300-410 m²/m³, a density of 1.6-2.1 g/cm³, and a diameter of 150-250 mm.

6. The device of claim 1, **characterized in that** micron calcium silicate in the micron calcium silicate tank has a particle size of 10-100 µm and a density of 2.9-3.1 g/cm³.

7. The device of claim 1, **characterized in that** the combined filler (9) has a specific surface area of 350-460 m²/m³, a density of 1.2-1.8 g/cm³, and a diameter of 150-250 mm; the fixed soft filler (10) has a density of 1.02-1.1 g/cm³, a fiber bundle length of 60-100 mm, a distance between bundles of 30-45 mm, and a porosity greater than 99%.

8. A method for pharmaceutical wastewater treatment with high efficiency resource recovery and low energy consumption using the device of any one of claims 1-7, the method comprising:
S1: introducing pharmaceutical wastewater to the floatation tank (1), removing oil and suspended particles, and eliminating supernatant pollutants of the floatation tank (1) using the decontamination device (17); allowing the pharmaceutical wastewater to flow into the pH adjusting tank (2), measuring a pH value of the pharmaceutical wastewater, and opening, by the controller 190, the magnetic valves of the acid tank (3) or the base tank (4) to adjust the pH value of the pharmaceutical wastewater to be 6.5-8.5;
S2: introducing the pharmaceutical wastewater from the pH adjusting tank (2) to the enhanced hydrolysis acidification tank (5) via the first uniform water distributor (18), and adding micron calcium silicate to the enhanced hydrolysis acidification tank (5), so that calcium silicate particles are absorbed onto a surface of sludge floc and dissociated to yield volatile fatty acids, which enhance the acid production capacity of microorganisms, and calcium ions, which increase the stability of the sludge floc, wherein a mass ratio of an addition amount of the micron calcium silicate to the volatile fatty acids from an effluent of the enhanced hydrolysis acidification tank (5) is 0.06-0.12, and a hydraulic retention time of the pharmaceutical wastewater is 6-8 h, and the fixed filler (7) in the middle part of the enhanced hydrolysis acidification tank is configured to intercept microorganisms and suspended particles in the pharmaceutical wastewater to ensure stable and efficient hydrolysis and acidification;
S3: introducing 5-10% of hydrolytic acidizing fluid from the enhanced hydrolysis acidification tank (5) to the anoxic pool (11) as a carbon source for denitrification, introducing rest of the hydrolytic acidizing fluid to the methanogenic tank (8), collecting and treating biogas produced in the methanogenic tank by the biogas collection and treatment device (13), and transporting the biogas as an energy source to sewage treatment plants, wherein a hydraulic retention time in the methanogenic tank (8) is 15-20 h, a sludge retention time is 20-30 d, calcium ions contained in an effluent of the enhanced hydrolytic acidification tank (5) is beneficial to the formation of granular sludge in the methanogenic tank, methanogenic bacteria is adsorbed on the combined filler (9) in the middle of the methanogenic tank (8) to form a biofilm composed of the methanogenic bacteria; the fixed soft filler (10) in the upper part of the methanogenic tank can intercept the methanogenic bacteria washed out, improving the methane production capacity and stability of the methanogenic tank (8); and
S4: introducing the wastewater from the methanogenic tank (8) to the anoxic pool (11), returning an effluent from the secondary sedimentation tank (14) to the anoxic pool (11), adding a suspension filler having a density of 0.93-0.96 g/cm³, a specific surface area of 400-460 m²/m³, and a diameter of 10-15 mm to the anoxic pool (11) to enrich denitrifying bacteria which adapts to reduce nitrate nitrogen to nitrogen, further introducing the wastewater to the aerobic pool (12) to remove chemical oxygen demand (COD) and ammonia nitrogen, separating the sludge and the water in the secondary sedimentation tank (14), adding polyaluminium chloride (PAC) to the coagulation sedimentation tank (15) for coagulating sedimentation, to yield final effluent.

## Patentansprüche

1. Vorrichtung zur pharmazeutischen Abwasserbehandlung mit hocheffizienter Ressourcenrückgewinnung und geringem Energieverbrauch, **dadurch gekennzeichnet, dass** sie Folgendes umfasst:
einen Flotationstank (1);
einen pH-Einstelltank (2);
einen Säuretank (3);
einen Basentank (4);
einen Tank für verstärkte Hydrolysensäuerung (5);
einen Mikroncalciumsilikattank (6);
einen methanogenen Tank (8);
ein anoxisches Becken (11);
ein aerobes Becken (12);
eine Biogassammel- und Behandlungsvorrichtung (13);
einen Nachklärtank (14);
einen Koagulations-Sedimentationstank (15);
einen Polyaluminiumchlorid (PAC)-Tank (16);
eine Steuervorrichtung (19) und
eine Stromversorgung;
wobei:
der Flotationstank (1), der pH-Einstelltank (2), der Tank für verstärkte Hydrolysensäuerung (5), der methanogene Tank (8), das anoxische Becken (11), das aerobe Becken (12), der Nachklärtank (14) und der Koagulations-Sedimentationstank (15) sequentiell verbunden sind;
eine Dekontaminationsvorrichtung (17) im Flotationstank (1) angeordnet ist, die es ermöglicht, überstehende Verunreinigungen im Flotationstank zu beseitigen;
der Säuretank (3) und der Basentank (4) unabhängig voneinander an den pH-Einstelltank (2) angeschlossen sind und ihre Anschlüsse jeweils mit Magnetventilen versehen sind;
der pH-Einstelltank (2) mit einem pH-Meter (21) versehen ist, und das pH-Meter (21) eine Messsonde umfasst, die im pH-Einstelltank (2) angeordnet ist;
der Tank für verstärkte Hydrolysensäuerung (5) einen oberen Teil, einen mittleren Teil und einen unteren Teil umfasst; der obere Teil an das anoxische Becken (11) über ein Saugrohr angeschlossen ist; ein befestigter Füller (7) im mittleren Teil angeordnet ist, und ein erster gleichmäßiger Wasserverteiler (18) im unteren Teil angeordnet ist;
der erste gleichmäßige Wasserverteiler (18) ein Hauptrohr (180) und ein Nebenrohr (181) umfasst, das im Hauptrohr (180) angeordnet ist; das Nebenrohr (181) ein Durchgangsloch umfasst, das mit der Kammer des Hauptrohrs (180) in Verbindung steht;
ein Drehmotor (182) im unteren Teil des Tanks für verstärkte Hydrolysensäuerung (5) angeordnet ist, um das Hauptrohr (180) mit Strom zu versorgen; das Hauptrohr (180) eine Hauptdüse (183) umfasst; und das Nebenrohr (181) eine Nebendüse (184) umfasst;
der Mikroncalciumsilikattank (6) an einen Boden des Tanks für verstärkte Hydrolysensäuerung (5) über ein Führungsrohr angeschlossen ist;
ein zweiter gleichmäßiger Wasserverteiler (18) in einem Boden des methanogenen Tanks (8) angeordnet ist; ein kombinierter Füller (9) in einem mittleren Teil des methanogonen Tanks (8) angeordnet ist, und ein befestigter weicher Füller (10) in einem oberen Teil des methanogenen Tanks (8) angeordnet ist;
das anoxische Becken (11) mit einem Suspensionsfüller gefüllt ist;
der Nachklärtank (14) an das anoxische Becken (11) über ein Rohr angeschlossen ist;
der PAC-Tank (16) an den Koagulations-Sedimentationstank (15) angeschlossen ist;
die Steuervorrichtung (19) einen Kontroller (190), einen Prozessor (191) und eine Fernbedienung (192) umfasst;
der Kontroller (190) an die Dekontaminationsvorrichtung (17), den Drehmotor (182), das pH-Meter (21) und die Magnetventile angeschlossen ist; und
der Prozessor (191) und die Fernbedienung (192) an den Kontroller (190) angeschlossen sind; und
die Stromversorgung die Dekontaminationsvorrichtung (17), den Drehmotor (182), das pH-Meter (21) und die Magnetventile mit Strom versorgt.

2. Vorrichtung nach Anspruch 1, **dadurch gekennzeichnet, dass:**
die Dekontaminationsvorrichtung (17) Dekontaminationsplatten (170), zwei Teleskopstangen (171), einen ersten Motor (172) und einen zweiten Motor (173) umfasst;
die zwei Teleskopstangen (171) jeweils an zwei inneren Enden des Flotationstanks (1) angeordnet sind;
zwei Gleitblöcke (174) jeweils an unteren Enden der zwei Teleskopstangen (171) angeordnet sind;
die zwei Gleitblöcke (174) über einen Verbindungsstab verbunden sind; zwei Enden des Verbindungsstabs jeweils mit Aufhängeschlaufen versehen sind;
obere Enden der zwei Teleskopstangen (171) jeweils mit Klemmplatten (175) versehen sind; und zwei Enden von jeder Dekontaminationsplatte (170) an die Klemmplatten (175) über drehbare Wellen flexibel angeschlossen sind;
eine Gleitbahn (176) in einer Bodenwand des Flotationstanks (1) angeordnet ist, und die zwei Teleskopstangen (171) an den Flotationstank (1) über die Gleitbahn (176) und die zwei Gleitblöcke (174) angeschlossen sind;
Riemenscheiben (177) an beiden Bodenenden des Flotationstanks (1) angeordnet sind; der erste Motor (172) an einem Bodenende des Flotationstanks (1) angeordnet ist und mit einem ersten Kabel versehen ist; das erste Kabel sich um eine der Riemenscheiben (177) an einem unteren Ende des Flotationstanks (1) wickelt und mit einer der Aufhängeschlaufe an einem Ende des Verbindungsstabs verbunden ist; und
der zweite Motor (173) an dem anderen unteren Ende des Flotationstanks (1) angeordnet ist und mit einem zweiten Kabel versehen ist; und das zweite Kabel sich um die andere Riemenscheibe (177) am anderen unteren Ende des Flotationstanks (1) wickelt und mit der anderen Aufhängeschlaufe am anderen Ende des Verbindungsstabs verbunden ist.

3. Vorrichtung nach Anspruch 2, **dadurch gekennzeichnet, dass** eine Kugel an einem Boden des Gleitblocks (174) angeordnet ist.

4. Vorrichtung nach Anspruch 2, **dadurch gekennzeichnet, dass** fünf Dekontaminationsplatten (170) verwendet werden, die parallel angeordnet sind und von den Klemmplatten (175) flexibel festgeklemmt werden; die Klemmplatten (175) jeweils mit einem befestigten Schenkel (178) versehen sind, und eine Vielzahl von linearen Aktuatoren (179) flexibel zwischen dem befestigten Schenkel (178) und den fünf Dekontaminationsplatten (170) angeordnet ist.

5. Vorrichtung nach Anspruch 1, **dadurch gekennzeichnet, dass** der befestigte Füller (7) eine spezifische Oberfläche von 300 - 410 m²/m³, eine Dichte von 1,6 - 2,1 g/cm³ und einen Durchmesser von 150- 250 mm aufweist.

6. Vorrichtung nach Anspruch 1, **dadurch gekennzeichnet, dass** das Mikroncalciumsilikat im Mikroncalciumsilikattank eine Partikelgröße von 10 - 100 µm und eine Dichte von 2,9 - 3,1 g/cm³ aufweist.

7. Vorrichtung nach Anspruch 1, **dadurch gekennzeichnet, dass** der kombinierte Füller (9) eine spezifische Oberfläche von 350 - 460 m²/m³, eine Dichte von 1,2 - 1,8 g/cm³ und einen Durchmesser von 150 - 250 mm aufweist; der befestigte weiche Füller (10) eine Dichte von 1,02 - 1,1 g/cm³, eine Faserbündellänge von 60 - 100 mm, einen Abstand zwischen Bündeln von 30 - 45 mm und eine Porösität von über 99 % aufweist.

8. Verfahren zur pharmazeutischen Abwasserbehandlung mit hocheffizienter Ressourcenrückgewinnung und geringem Energieverbrauch unter Einsatz der Vorrichtung nach einem der Ansprüche 1 - 7 , wobei das Verfahren Folgendes umfasst:
S1: Einleiten von pharmazeutischem Abwasser in den Flotationstank (1), Entfernen von Öl und suspendierten Partikeln und Beseitigen von überstehenden Verunreinigungen des Flotationstanks (1) unter Anwendung der Dekontaminationsvorrichtung (17); Fließenlassen des pharmazeutischen Abwassers in den pH-Einstelltank (2), Messen eines ph-Werts des pharmazeutischen Abwassers und Öffnen der Magnetventile des Säuretanks (3) oder des Basentanks (4) mit der Steuerung 190, um den pH-Wert des pharmazeutischen Abwassers auf 6,5 - 8,5 einzuregeln;
S2: Einleiten des pharmazeutischen Abwassers vom pH-Einstelltank (2) in den Tank für verstärkte Hydrolysensäuerung (5) über den ersten gleichförmigen Wasserverteiler (18), und Zugeben von Mikrocalciumsilikat zum Tank für verstärkte Hydrolysensäuerung (5), sodass Calciumsilikatpartikel auf eine Oberfläche der Schlammflocke adsorbiert und dissoziiert wird, um flüchtige Fettsäuren zu erhalten, welche die Säureproduktionskapazität von Mikroorganismen erhöhen, und Calciumionen, welche die Stabilität der Schlammflocke verstärken, wobei das Masseverhältnis einer Zusatzmenge von Mikrocalciumsilikat zu den flüchtigen Fettsäuren aus einem Abfluss des Tanks für verstärkte Hydrolysensäuerung (5) 0,06 - 0,12 ist, und eine hydraulische Verweilzeit des pharmazeutischen Abwassers 6 - 8 Stunden beträgt, und der befestigte Füller (7) im mittleren Teil des Tanks für verstärkte Hydrolosensäuerung konfiguriert ist, um Mikroorganismen und suspendierte Partikel im pharmazeutischen Abwasser abzufangen, um eine stabile und effiziente Hydrolyse und Säuerung sicherzustellen;
S3: Einleiten von 5 - 10 % der hydrolytischen Säuerungsflüssigkeit vom Tank für verstärkte Hydrolysensäuerung (5) in das anoxische Becken (11) als eine Kohlenstoffquelle für die Denitrifizierung, Einleiten des Rests der hydrolytischen Säuerungsflüssigkeit in den methanogenen Tank (8), Sammeln und Behandeln des im methanogenen Tank produzierten Biogases durch die Biogassammel- und Behandlungsvorrichtung (13) und Transportieren des Biogases als eine Energiequelle zu Kläranlagen, wobei eine hydraulische Verweilzeit im methanogenen Tank (8) 15 - 20 Stunden und eine Schlammverweilzeit 20 - 30 Tage beträgt, Calciumionen, die in einem Abfluss des Tanks für verstärkte Hydrolysensäuerung (5) vorteilhaft für die Bildung von körnigem Schlamm im methanogenen Tank sind, methanogene Bakterien auf den kombinierten Füller (9) in der Mitte des methanogenen Tanks (8) adsorbiert werden, um einen Biofilm zu bilden, der aus methanogenen Bakterien besteht; der befestigte weiche Füller (10) im oberen Teil des methanogenen Tanks die ausgewaschenen methanogenen Bakterien auffangen kann, wodurch die Methanproduktionskapazität und -stabilität des methanogenen Tanks (8) verbessert wird; und
S4: Einleiten des Abwassers aus dem methanogenen Tank (8) in das anoxische Becken (11), Rückführen eines Abflusses vom Nachklärbecken (14) in das anoxische Becken (11), Zugeben eines Suspensionsfüllers, der eine Dichte von 0,93 - 0,96 g/ cm³, eine spezifische Oberfläche von 400 - 460 m²/m³ und einen Durchmesser von 10 - 15 mm aufweist, in ein anoxisches Becken (11), um denitrifizierende Bakterien anzureichern, die geeignet sind, um Nitratstickstoff zu Stickstoff abzubauen, ferner Einleiten des Abwassers in das aerobe Becken (12), um chemischen Sauerstoffbedarf (CSB) aufzuheben und Ammoniakstickstoff zu entfernen, Trennen von Schlamm und Wasser im Nachklärbecken (14), Zugeben von Polyaluminiumchlorid (PAC) zum Koagulations-Sedimentationstank (15) zur koagulierenden Sedimentation, um das endgültige Abwasser zu erhalten.

## Revendications

1. Dispositif pour le traitement d'eaux usées pharmaceutiques avec une récupération de ressources de haute efficacité et une faible consommation d'énergie, caractérisé comme comprenant :
une cuve de flottaison (1) ;
une cuve d'ajustement de pH (2) ;
une cuve d'acide (3) ;
une cuve de base (4) ;
une cuve d'acidification d'hydrolyse améliorée (5) ;
une cuve de silicate de calcium micronique (6) ;
une cuve méthanogène (8) ;
un bassin anoxique (11) ;
un bassin aérobie (12) ;
un dispositif de collecte et de traitement de biogaz (13) ;
une cuve de sédimentation secondaire (14) ;
une cuve de sédimentation par coagulation (15) ;
une cuve de chlorure de polyaluminium (PAC) (16) ;
un dispositif de commande (19) ; et
une alimentation de puissance ;:
où:
la cuve de flottaison (1), la cuve d'ajustement de pH (2), la cuve d'acidification d'hydrolyse améliorée (5), la cuve méthanogène (8), le bassin anoxique (11), le bassin aérobie (12), la cuve de sédimentation secondaire (14) et la cuve de sédimentation par coagulation (15) étant connectées séquentiellement ;
un dispositif de décontamination (17) permettant d'éliminer les polluants de surnageant de la cuve de flottaison étant disposé dans la cuve de flottaison (1) ;
la cuve d'acide (3) et la cuve de base (4) étant connectées indépendamment à la cuve d'ajustement de pH (2), et leurs joints étant fournis avec des vannes magnétiques, respectivement ;
la cuve d'ajustement de pH (2) étant fournie avec un pH mètre (21), et le pH mètre (21) comprenant une sonde de mesure disposée dans la cuve d'ajustement de pH (2) ;
la cuve d'acidification d'hydrolyse améliorée (5) comprenant une partie supérieure, une partie moyenne et une partie inférieure ; la partie supérieure étant connectée au bassin anoxique (11) via un tube provisoire ; un remplisseur fixé (7) étant disposé dans la partie moyenne et un premier distributeur d'eau uniforme (18) étant disposé dans la partie inférieure ;
le premier distributeur d'eau uniforme (18) comprenant un tube principal (180) et un tube secondaire (181) disposé dans le tube principal (180) ; le tube secondaire (181) comprenant un trou communiquant avec une chambre du tube principal (180) ;
un moteur rotatif (182) étant disposé dans la partie inférieure de la cuve d'acidification d'hydrolyse améliorée (5) pour fournir de la puissance pour le tube principal (180) ; le tube principal (180) comprenant une buse principale (183) et le tube secondaire (181) comprenant une buse secondaire (184) ;
la cuve de silicate de calcium micronique (6) étant connectée à une partie inférieure de la cuve d'acidification d'hydrolyse améliorée (5) via un tube guide ;
un second distributeur d'eau uniforme (18) étant disposé dans une partie inférieure de la cuve méthanogène (8) ; un remplisseur combiné (9) étant disposé dans une partie moyenne de la cuve méthanogène (8) et un remplisseur mou fixé (10) étant disposé dans une partie supérieure de la cuve méthanogène (8)
le bassin anoxique (11) étant rempli avec un remplisseur de suspension ;
la cuve de sédimentation secondaire (14) étant connectée au bassin anoxique (11) via un tuyau ;
la cuve PAC (16) étant connectée à la cuve de sédimentation par coagulation (15);
le dispositif de commande (19) comprenant un contrôleur (190), un processeur (191) et une télécommande(192) ;
le contrôleur (190) étant connecté au dispositif de décontamination (17), au moteur rotatif (182), au pH mètre (21) et aux vanne magnétiques ;
le processeur (191) et la télécommande (192) étant connectés au contrôleur (190) ; et
l'alimentation de puissance fournissant de la puissance pour le dispositif de décontamination (17), le moteur :rotatif (182), le pH mètre (21) et les vannes magnétiques.

2. Dispositif selon la revendication 1, **caractérisé en ce que** :
le dispositif de décontamination (17) comprend des panneaux de décontamination (170), deux tiges télescopiques (171), un premier moteur (172) et un second moteur (173) ;
les deux tige télescopiques (171) sont disposées à deux extrémités internes de la cuve de flottaison (1), respectivement ;
deux blocs glissants (174) sont disposés respectivement à des extrémités inférieures des deux tiges télescopiques (171) ;
les deux blocs glissants (174) sont connectés via une tige de connexion ; deux extrémités de la tige de connexion sont fournies avec des boucles de suspension, respectivement ;
les extrémités supérieures des deux tiges télescopiques (171) sont fournies avec des plaques de serrage (175), respectivement ; et deux extrémités de chaque panneau de décontamination (170) sont connectées de manière flexible aux plaques de serrage (175) via des arbres tournants ;
une glissière (176) est disposée dans une paroi de partie inférieure de la cuve de flottaison (1) et les deux tiges télescopiques (171) sont connectées à la cuve de flottaison (1) via la glissière (176) et les deux blocs glissants (174) ;
des poulies (177) sont disposées à deux extrémités de la partie inférieure de la cuve de flottaison (1) ; le premier moteur (172) est disposé à une extrémité de partie inférieure de la cuve de flottaison (1) et est fourni avec un premier câble ; le premier câble s'enroule autour de l'une des poulies (177) à une extrémité de partie inférieure de la cuve de flottaison (1) et se connecte à une des boucles de suspension à une extrémité de la tige de connexion ; et
le second moteur (173) est disposé à l'autre extrémité de partie inférieure de la cuve de flottaison (1) et est fourni avec un second câble ; et le second câble s'enroule autour de l'autre poulie (177) à l'autre extrémité de partie inférieure de la cuve de flottaison (1) et se connecte à l'autre boucle de suspension à l'autre extrémité de la tige de connexion.

3. Dispositif selon la revendication 2, **caractérisé en ce qu'**une boule est disposée à une partie inférieure des blocs glissants (174).

4. Dispositif selon la revendication 2, **caractérisé en ce que** cinq panneaux de decontamination (170) sont employés, disposés en parrallèle, et serrés de manière flexible par les plaques de serrage (175); les plaques de serrage (175) sont fournies avec une jambe fixée (178) et une pluralité d'organes de commande linéaires (179) sont disposés de manière flexible entre la jambe fixée (178) et les cinq panneaux de décontamination (170).

5. Dispositif selon la revendication 1, **caractérisé en ce que** le remplisseur fixé (7) a une surface spécifique de 300 à 410 m²/m³, une densité de 1,6 à 2,1 g/cm³ et un diamètre de 150 à 250 mm.

6. Dispositif selon la revendication 1, **caractérisé en ce que** le silicate de calcium micronique dans la cuve de silicate de calcium micronique a une taille de particule de 10 à 100 µm et une densité de 2,9 à 3,1 g/cm³.

7. Dispositif selon la revendication 1, **caractérisé en ce que** le remplisseur combiné (9) a une surface spécifique de 350 à 460 m²/m³, une densité de 1,2 à 1,8 g/cm³ et un diamètre de 150 à 250 mm ; le remplisseur mou fixé (10) a une densité de 1,02 à 1,1 g/cm³, une longueur de faisceau de fibres de 60 à 100 mm, une distance entre deux faisceaux de 30 à 45 mm et une porosité supérieure à 99 %.

8. Procédé pour le traitement des eaux usées pharmaceutiques avec une récupération de resources de haute efficacité et une faible consommation d'énergie en utilisant le dispositive selon l'une quelconque des revendications 1 à 7, le proceed comprenant les étapes consistant à:
S1: introduire les eaux usées pharmaceutiques dans la cuve de flottaison (1), enlever l'huile et les particules en suspension, et éliminer les polluants de surnageant de la cuve de flottaison (1) en utilisant le dispositif de décontamination (17) ; permettre aux eaux usées pharmaceutiques de s'écouler dans la cuve d'ajustement de pH (2), mesurer une valeur d pH des eaux uses pharmaceutiques, et ouvrir, par le contrôleur 190, les vannes magnétiques de la cuve d'acide (3) ou de la cuve de base (4) pour ajuster la valeur de pH des aux usées pharmaceutiques à 6,5 à 8,5 ;
S2: introduire les eaux usées pharmaceutiques de la cuve d'ajustement de pH (2) dans la cuve d'acidification d'hydrolyse améliorée (5) via le premier distributeur d'eau uniforme (18), et ajouter le silicate de calcium micronique à la cuve d'acidification d'acide améliorée (5), de sorte que les particules de silicate de calcium sont absorbées sur une surface de boue activée et dissociées pour donner de acides gras volatils, qui augmentent la capacité de production d'acide des microorganismes, et des ions calcium, qui augmentent la stabilité de la boue activée, un rapport en masse d'une quantité d'addition du silicate de calcium micronique aux acides gras volatils d'un effluent de la cuve d'acidification d'hydrolyse améliorée (5) est de 0,06 à 0,12 et un temps de rétention hydraulique des eaux usées pharmaceutiques est de 6 à 8 heures, et le remplisseur fixé (7) dans la partie de milieu de la cuve d'acidification d'hydrolyse améliorée est configuré pour intercepter les microorganismes et les particules en suspension dans les eaux usées pharmaceutiques pour assurer une hydrolyse et une acidification stables et efficaces ;
S3: introduire 5 à 10 % de fluide acidifiant hydrolytique de la cuve d'acidification d'hydrolyse améliorée (5) dans le bassin anoxique (11) comme source de carbone pour la dénitrification, introduire le reste du fluide acidifiant hydrolytique dans la cuve méthanogène (8), collecter et traiter le biogaz produit dans la cuve méthanogène par le dispositif de collecte et de traitement de biogaz (13), et transporter le biogaz comme source d'énergie à des usines de traitement d'eaux usées, un temps de rétention hydraulique dans la cuve méthanogène (8) étant de 15 à 20 heures, un temps de rétention de boue étant de 20 à 30 jours, les ions calcium contenus dans un effluent de la cuve d'acidification hydrolytique améliorée (5) étant bénéfiques pour la formation de boue granulaire dans la cuve méthanogène, les bactéries méthanogènes étant adsorbées sur le remplisseur combiné (9) dans le milieu de la cuve méthanogène (8) pour former un biofilm composé des bactéries méthanogènes ; le remplisseur mou fixé (10) dans la partie supérieure de la cuve méthanogène pouvant intercepter les bactéries méthanogènes éliminées par lavage, améliorant la capacité de production et la stabilité du méthane de la cuve méthanogène (8) ; et
S4: introduire les eaux usées de la cuve méthanogène (8) dans le bassin anoxique (11), retournant un effluent de la cuve de sédimentation secondaire (14) au bassin anoxique (11), ajouter un remplisseur de suspension ayant une densité de 0,93 à 0,96 g/cm³, une surface spécifique de 400 à 460 m²/m³ et un diamètre de 10 à 15 mm au bassin anoxique (11) pour enrichir les bactéries dénitrifiantes qui s'adaptent à réduire l'azote du nitrate en azote, encore introduire les eaux usées dans le bassin aérobie (12) pour enlever la demande d'oxygène chimique (COD) et l'azote de l'ammoniaque, séparer la boue et l'eau dans la cuve de sédimentation secondaire (14), ajouter le chlorure de polyaluminium (PAC) à la cuve de sédimentation par coagulation (15) pour la sédimentation par coagulation, pour donner l'effluent final.
